# EUROPÄISCHE PATENTSCHRIFT

(11) **EP 0 770 625 B1**
(45) Veröffentlichungstag und Bekanntmachung des Hinweises auf die Patenterteilung: **24.07.2002**
(21) Anmeldenummer: 96115218.8
(22) Anmeldetag: 23.09.1996
(51) Int. Cl.: C07K 14/745, C07K 1/22, C12N 9/64

(54) **Verfahren zur Reinigung von Faktor VII und aktiviertem Faktor VII**
Process for the purification of Factor VII and activated Factor VII
Procédé de purification de Facteur VII et Facteur VII activé

(30) Priorität: 18.10.1995 DE 19538715
(43) Veröffentlichungstag der Anmeldung: 02.05.1997
(73) Patentinhaber: Aventis Behring Gesellschaft mit beschränkter Haftung, 35002 Marburg (DE)
(72) Erfinder: Römisch, Jürgen, Dr., 35041 Marburg (DE); Stöhr, Hans-Arnold, 35083 Wetter (DE); Feussner, Annette, 35043 Marburg (DE)

(56) Entgegenhaltungen:
- EP-A- 0 346 241
- EP-A- 0 464 533
- EP-A- 0 547 932
- WO-A-94/22905
- KANG S ET AL: "PURIFICATION OF HUMAN BRAIN TISSUE FACTOR." THROMB HAEMOSTASIS 59 (3). 1988. 400-403, XP002063459
- CHEMICAL ABSTRACTS, vol. 107, no. 21, 23.November 1987 Columbus, Ohio, US; abstract no. 194493, RAO, L. VIJAYA MOHAN ET AL: "Affinity purification of human brain tissue factor utilizing factor VII bound to immobilized anti- factor VII" XP002063460 & ANAL. BIOCHEM. (1987), 165(2), 365-70,
- MORRISSEY J.H.: 'Tissue Factor Modulation of Factor VIIa Activity: Use in Measuring Trace Levels of Factor VIIa in Plasma' THROMBOSIS AND HAEMOSTASIS Bd. 74, Nr. 1, Juli 1995, GERMANY, Seiten 185 - 188
- KELLEY R.F. ET AL.: 'Analysis of the factor VIIa binding site on human tissue factor: effects of tissue factor mutations on the kinetics and thermodynamics of binding' BIOCHEMISTRY Bd. 34, Nr. 33, 22 August 1995, Seiten 10383 - 10392

## Beschreibung

Die Erfindung betrifft ein Verfahren zur Reinigung von Faktor VII und/oder aktiviertem Faktor VII (F VII/F Vlla) mittels Bindung an immobilisiertes lösliches Thromboplastin.

Gerinnungsfaktor VII (FVII) stellt zusammen mit dem Thromboplastin (Tissue Factor, TF) den Initiatorkomplex des exogenen Gerinnungsweges dar. Bei Gewebeverletzung wird TF exponiert und ermöglicht die Bindung von FVII und/oder FVIIa an dessen extrazelluläre Proteindomäne. Ein hydrophober Proteinbereich verankert TF in der Membran.

Der physiologisch aktive Anteil des FVII/FVIIa-Gemisches, nämlich TF-FVIIa, aktiviert in Gegenwart von (bevorzugt negativ geladenen) Lipiden und Kalzium effektiv den Faktor X (FX). FXa wiederum katalysiert (zusammen mit FVa, Lipiden und Kalzium) die Generierung von Thrombin. Die anschließende Bildung von Fibrin stellt (u.a.) einen Wundverschluß sicher.

FVII, gebunden an membranständigen TF, wird wiederum durch Autoaktivierung (TF-FVIIa-vermittelt) oder durch FIXa, FXa und Thrombin aktiviert, wodurch die kaskadenförmige Aktivierung des Gerinnungssystemes weiter verstärkt wird.

Ein Mangel an FVII kann entsprechend mit hämostatischen Komplikationen, wie Blutungsneigung, einhergehen. Als ein Gerinnungsfaktor, dessen Synthese zum physiologisch funktionsfähigen Molekül von der Anwesenheit von Vitamin K abhängt, können Komplikationen entsprechend z.B. bei oraler Antikoagulation (mit Vitamin K-Antagonisten) vor Operationen auftreten; weitere Indikationen zur Substitution mit FVII stellen Verbrauchskoagulopathien oder Leberschäden dar. FVIIa dagegen ist Bestandteil von aktivierten PPSB-Präparaten, die bei akuten Blutungen Verwendung finden können. Darüber hinaus besitzt FVIIa eine sog. Faktor VIII "Bypassing" Aktivität, die bei der Substitution von Hämophilen mit z.B. FVIII-Unverträglichkeit (z.B. Antikörper) genutzt wird.

FVII/FVIIa wird üblicherweise mittels mehrerer Präparationsschritte aus Plasma oder Kulturüberständen (bei rekombinanter Herstellung) angereichert, was meist mit entsprechenden Ausbeuteverlusten einhergeht. Als ein Protein, das strukturell und in seinen Eigenschaften mit anderen Gerinnungsfaktoren verwandt ist, ist die Reinigung aus einem entsprechenden Gemisch schwierig und aufwendig. Im Falle von FVII besteht darüber hinaus die Gefahr, daß mit zunehmendem Aufwand des Reinigungsvorganges eine Aktivierung eintritt (zu FVIIa), die (entsprechend den angestrebten Präparaten) vermieden werden muß. Schnelle Verfahren stellen z.B. Präparationen mittels geeigneter immobilisierter monoklonaler Antikörper dar. Meistens sind jedoch Bedingungen zur Elution nötig (pH 3-pH 4), die das Protein irreversibel schädigen. Partielle Denaturierung führt entsprechend zu Ausbeuteverlusten und kann auch eine Antigenität der konformationell geänderten Molekülstrukturen verursachen.

Der vorliegenden Erfindung liegt daher die Aufgabe zugrunde, ein Verfahren zur schnellen und schonenden Reinigung von FVII/FVIIa bereitzustellen.

Die Aufgabe wurde wie folgt gelöst: Aus einer FVII/FVIIa-haltigen Lösung werden FVII und/oder FVIIa an einen immobilisierten sTF ("lösliches" Thromboplastin, soluble Tissue Factor) gebunden, nicht gebundene Moleküle durch Waschen der Matrix entfent und FVII/FVIIa anschließend schonend eluiert. sTF ist hierbei Thromboplastin ohne den Transmembrananteil und den cytoplasmatischen Anteil, also die extrazelluläre Domäne von TF.

Wir haben gefunden, daß zur Reinigung von FVII und/oder FVIIa die Interaktion von FVII/VIIa mit sTF genutzt werden kann. Während nämlich der "physiologische" komplette (Lipid bindende) TF die Autoaktivierung und die Proteolyse bzw. Rückaktivierung des gebundenen FVII durch FIXa, FXa und Thrombin vermittelt, bleibt an sTF gebundener FVII unversehrt (Morrissey; Thromb. Haemostas. 1995; 74:185-188).

Die Bindung von FVII und/oder FVIIa an sTF wird in Gegenwart zweiwertiger Ionen optimiert, besonders durch Kalzium, während andere Proteine ungebunden entfernt werden können. Entsprechend wird ein immobilisierter sTF an eine Matrix adsorbiert, aus einer Lösung in Gegenwart von Kalzium FVII und/oder FVIIa an sTF gebunden und ungebundene Moleküle durch Waschen der Festphase entfernt. Die Elution wird schonend mittels eines Puffers, der einen Chelatbildner enthält, wie z.B: Citrat, Oxalat, Tartrat, EDTA o.Ä., eluiert.

Der sTF kann über nicht-kovalente Bindung an eine Festphase adsorbiert oder durch kovalente Bindung immobilisiert werden. Als Bindungspartner kommen herkömmlich präparierter sTF (proteolytisch hergestellt aus komplettem TF), rekombinant hergestellter sTF oder FVII/VIIa- bindende Anteile (Peptidbereiche aus sTF) in Frage. Der sTF bzw. entsprechende FVII-Bindungsregionen können an Substanzen gekoppelt sein, die eine Immobilisierung vereinfachen bzw. optimieren (z.B. als 'Spacer'-Funktion, s.u.).

In einer bevorzugten Verfahrensweise wird ein an ein Fc-Fragment gekoppelter sTF (Fc-sTF) verwendet, wie in EP 464533 (Lauffer et al) beschrieben. Durch Bindung des Fc-sTF z.B. an eine Anti-Fc-Säule oder eine Protein A- oder -G-Matrix wird der sTF optimal präsentiert und die Effizienz des Reinigungssystemes gesteigert. Da Proben, die Fc-Fragmente oder Immunglobuline enthalten, zu einer Verdrängung des Fc-sTF von der Matrix führen können, empfiehlt sich die kovalente Kopplung des Fc-sTF an die Matrix mittels bekannter Methoden.

Der FVII/FVIIa- haltigen Lösung werden zweiwertige Ionen, bevorzugt Kalzium, bevorzugt in Form von CaCl2, in einer Konzentration von 0,01 bis 500 mM, besonders bevorzugt 0,5 bis 50 mM, zugesetzt. Die Lösung sollte einen pH-Wert von 5,0 bis 10,0 haben, bevorzugt 7,0 bis 9,5. Diese Lösung wird mit der sTF-Matrix in Kontakt gebracht, die Matrix mit Pufferlösung gewaschen, die bevorzugt einen pH-Wert zwischen 7,0 und 9,5 und eine Kalzium-Konzentration von 0,5 und 50 mM aufweist. Die Elution erfolgt durch eine Lösung die einen Chelatbildner enthält, bevorzugt Citrat, Oxalat, Tartrat, NTA, EDTA oder EGTA in Konzentrationen von 0,1-1000 mM, bevorzugt zwischen 5 und 200 mM. Die Lösung hat einen pH-Wert von 5,0 bis 10,0, bevorzugt 5,5 bis 8,5, besonders bevorzugt 6,0 bis 7,5.

Proben, die aktivierte Faktoren enthalten, können zu einer weiteren Generierung von FVIIa (ggfs. aus dem Überschuß des präsenten Faktors VII) und damit zu artifiziellen Ergebnissen führen. Zur Vorbeugung dieses Risikos kann der Probe vor Kontakt mit dem immobilisierten sTF Antithrombin III/Heparin zugesetzt werden. Da FVIIa durch ATIII/Hep. bei Raumtemperatur bzw. höheren Temperaturen im Vergleich zu potentiell störenden anderen Faktoren (Flla, FIXa, FXa etc.) nur langsam inhibiert wird, können die letzteren blockiert werden, ohne den Gehalt an FVIIa wesentlich zu beeinflussen, falls eine Reinigung von FVIIa beabsichtigt ist. TF-gebundener FVIIa kann u.U. durch ATIII/Hep. besser inhibierbar sein als die freien Moleküle (ATIII reagiert jedoch ohne funktionelles Heparin nur sehr langsam, analog löslichem FVIIa). Daher kann vor Kontakt mit sTF das zugesetzte Heparin mittels bekannter Reagenzien wie Protaminsulfat/Polybren® neutralisiert werden und anschließend wie oben beschrieben verfahren werden.

Für diesen Verfahrensschritt eignen sich ebenfalls reversible Inhibitoren , z.B. Benzamidin, und andere Kofaktor-abhängige Inhibitoren, die selbst oder deren Akzeleratoren neutralisiert werden können (beispielsweise Heparin-Kofaktor II / Heparin).

Die Erfindung wird durch folgendes Beispiel näher erläutert:

### Beispiel:

Protein A-Sepharose wurde mit Fc-sTF beladen (10 µg/50 µl Gelmatrix) und mit Puffer A (50 mM Tris/HCl, 150 mM NaCI, 10 mM CaCl2, 0,1% Human-Albumin, pH: 8,5) äquilibriert. 0,5 ml einer Proteinlösung, die FVII (15 IE/ml) enthielt, wurde FVIIa zugesetzt auf eine Konzentration, die 5% (im Gerinnungstest) der FVII-Aktivität entsprach. Andere Gerinnungsfaktoren, wie die Faktoren II (30 IE/ml), IX (25 IE/ml) und X (30 IE/ml), sowie eine Anzahl weiterer Plasmaproteine, waren ebenfalls enthalten. Diese Lösung wurde mit dem gleichen Volumen Puffer A verdünnt und mit der sTF-Matrix in einer kleinen Säule in Kontakt gebracht. Nach Durchlaufen der Säule wurde die Matrix mit 0,5 ml Puffer A gewaschen und anschließend gebundenes Protein mit Puffer B (50 mM Tris/HCl, 150 mM NaCI, 50 mM Natrium-Citrat, pH 6,5) eluiert und aufgefangen.

Das Eluat wurde in entsprechenden Gerinnungstests geprüft und die Ausbeute an FVII/VIIa sowohl durch dessen Aktivität als auch mittels ELISA (in Bezug auf das Ausgangsmaterial) quantifiziert. Die Reinheit des Eluates wurde durch SDS-PAGE demonstriert.

### Ergebnis:

Die Ausbeute an FVII/VIIa betrug nach ELISA 93%, nach Aktivität 90%, bezogen auf das Ausgangsmaterial. Ein wichtiger Befund war ebenfalls, daß im Eluat wieder 5% der FVII-Aktivität von (dem zugesetzten) FVIIa stammte. Dies zeigt, daß nicht eines der beiden Moleküle bevorzugt gebunden wurde. Andererseits kann ausgeschlossen werden, daß während dieses Reinigungsschrittes eine Aktivierung von FVII zu FVIIa stattgefunden hat.

Im Eluat wurden weder FII, FIX noch FX gefunden, jedoch alles (entsprechend Ausgangsmaterial) im Säulendurchlauf. Die Reinheit des Eluates und der starke Anreicherungseffekt von FVII/VIIa im Eluat wird durch SDS-PAGE Analyse verdeutlicht, aus der der Reinigungseffekt überzeugend hervorgeht.

## Patentansprüche

1. Verfahren zur Reinigung von Faktor VII und/oder Faktor VII a, **dadurch gekennzeichnet, daß** aus einer Faktor VII und/oder Faktor VIIa-haltigen Lösung Faktor VII und/oder Faktor VII a an immobilisiertes lösliches Thromboplastin gebunden wird, nicht gebundene Moleküle durch Waschen entfernt werden und man den gebundenen Faktor VII und/oder Faktor VIIa eluiert.

2. Verfahren nach Anspruch 1, **dadurch gekennzeichnet, daß** mittels eines Chelatbildners gebundener Faktor VII und/oder Faktor VII a eluiert wird.

3. Verfahren nach Anspruch 1 oder Anspruch 2, **dadurch gekennzeichnet, daß** Ca ²⁺ Ionen in einer Konzentration zwischen 0,01 bis 500 mM zugesetzt und bei der Bindung an immobilisiertes lösliches Thromboplastin ein pH von 5,0 - 10,0 gewählt wird.

4. Verfahren nach Anspruch 2 oder Anspruch 3, **dadurch gekennzeichnet, daß** als Chelatbildner Citrat, Oxalat, Tartrat, NTA, EDTA oder EGTA in einer Konzentration von 0,1 - 1000 mM, ausreichend zur Elution von Faktor VII und/oder Faktor VII a gewählt wird.

5. Verfahren nach einem der vorhergehenden Ansprüche, **dadurch gekennzeichnet, daß** andere störende aktivierte Faktoren vor dem Kontakt mit immobilisiertem löslichen Thromboplastin inhibiert werden.

6. Verfahren nach Anspruch 5, **dadurch gekennzeichnet, daß** vor dem Kontakt mit immobilisiertem löslichen Thromboplastin potentiell störende andere aktivierte Faktoren durch Zugabe von Antithrombin III/Heparin inaktiviert werden und gegebenenfalls anschließend Protaminsulfat zugegeben wird.

7. Verfahren nach einem der vorhergehenden Ansprüche, **dadurch gekennzeichnet, daß** lösliches Thromboplastin über nicht kovalente Bindung an die Festphase adsorbiert wird.

8. Verfahren nach Anspruch 7, **dadurch gekennzeichnet, daß** ein an ein Fc-Fragment gekoppeltes lösliches Thromboplastin eingesetzt wird.

9. Verfahren nach Anspruch 1 bis Anspruch 6, **dadurch gekennzeichnet, daß** lösliches Thromboplastin kovalent an die Festphase gebunden wird.

## Claims

1. A process for purifying factor VII and/or factor Vila, which comprises removing factor VII and/or factor VIIa from a factor VII and/or factor VIIa-containing solution by binding them to immobilized soluble thromboplastin, removing unbound molecules by washing, and eluting the bound factor VII and/or factor VIIa.

2. The process as claimed in claim 1, wherein bound factor VII and/or factor Vila is eluted using a chelating agent.

3. The process as claimed in claim 1 or claim 2, wherein Ca²⁺ ions are added at a concentration of between 0.01 and 500 mM, and a pH of 5.0 - 10.0 is selected in association with the binding to immobilized soluble thromboplastin.

4. The process as claimed in claim 2 or claim 3, wherein citrate, oxalate, tartrate, NTA, EDTA or EGTA, at a concentration of 0.1 - 1000 mM, which is sufficient to elute factor VII and/or factor VIIa, is selected as the chelating agent.

5. The process as claimed in one of the preceding claims, wherein other, interfering activated factors are inhibited prior to the contact with immobilized soluble thromboplastin.

6. The process as claimed in claim 5, wherein, prior to the contact with immobilized soluble thromboplastin, other, potentially interfering activated factors are inactivated by adding antithrombin III/heparin, and protamine sulfate is subsequently added, where appropriate.

7. The process as claimed in one of the preceding claims, wherein soluble thromboplastin is adsorbed to the solid phase by way of non-covalent bonding.

8. The process as claimed in claim 7, wherein a soluble thromboplastin is employed which is coupled to an Fc fragment.

9. The process as claimed in claim 1 to claim 6, wherein soluble thromboplastin is covalently bonded to the solid phase.

## Revendications

1. Procédé pour la purification du facteur VII et/ou du facteur VIIa, **caractérisé en ce qu'**à partir d'une solution contenant du facteur VII et/ou du facteur VIIa, le facteur VII et/ou le facteur VIIa est(sont) fixé(s) à une thromboplastine soluble immobilisée, les molécules non fixées sont éliminées par lavage et on élue le facteur VII et/ou le facteur VIIa fixé(s).

2. Procédé selon la revendication 1, **caractérisé en ce qu'**on élue le facteur VII et/ou le facteur VIIa fixé(s) au moyen d'un agent chélateur.

3. Procédé selon la revendication 1 ou la revendication 2, **caractérisé en ce qu'**on ajoute des ions Ca²⁺ à une concentration comprise entre 0,01 et 500 mM et on choisit un pH de 5,0-10,0 lors de la liaison à la thromboplastine soluble immobilisée.

4. Procédé selon la revendication 2 ou la revendication 3, **caractérisé en ce que** comme agent chélateur on choisit un citrate, oxalate, tartrate, NTA, EDTA ou EGTA à une concentration de 0,1 - 1 000 mM, suffisante pour l'élution du facteur VII et/ou du facteur VIIa.

5. Procédé selon l'une quelconque des revendications précédentes **caractérisé en ce que** d'autres facteurs activés gênants sont inhibés avant le contact avec la thromboplastine soluble immobilisée.

6. Procédé selon la revendication 5, **caractérisé en ce que** d'autres facteurs activés potentiellement gênants sont inactivés, avant le contact avec la thromboplastine soluble immobilisée, par addition d'antithrombine III/héparine et éventuellement on ajoute ensuite du sulfate de protamine.

7. Procédé selon l'une quelconque des revendications précédentes, **caractérisé en ce que** la thromboplastine soluble est adsorbée sur la phase solide par liaison non covalente.

8. Procédé selon la revendication 7, **caractérisé en ce qu'**on utilise une thromboplastine soluble couplée à un fragment Fc.

9. Procédé selon la revendication 1 à la revendication 6, **caractérisé en ce que** la thromboplastine soluble est liée par covalence à la phase solide.
